# EUROPÄISCHE PATENTSCHRIFT

(11) **EP 1 527 153 B1**
(45) Veröffentlichungstag und Bekanntmachung des Hinweises auf die Patenterteilung: **28.02.2007**
(21) Anmeldenummer: 03784096.4
(22) Anmeldetag: 29.07.2003
(51) Int. Cl.: C11C 3/14, C07C 67/333

(54) **VERFAHREN ZUR HERSTELLUNG VON KONJUGIERTER LINOLS URE**
METHOD FOR THE PRODUCTION OF CONJUGATED LINOLEIC ACIDS
PROCEDE DE PREPARATION D'ACIDE LINOLEIQUE CONJUGUE

(30) Priorität: 07.08.2002 DE 10236086
(43) Veröffentlichungstag der Anmeldung: 04.05.2005
(73) Patentinhaber: Cognis IP Management GmbH, 40589 Düsseldorf (DE)
(72) Erfinder: WESTFECHTEL, Alfred, 40724 Hilden (DE); ALBIEZ, Wolfgang, 41468 Neuss (DE); BUSCH, Stefan, 46047 Oberhausen (DE); ZANDER, Lars, 40597 Düsseldorf (DE); HORLACHER, Peter, 89287 Bellenberg (DE)
(86) Internationale Anmeldenummer: PCT/EP2003/008339
(87) Internationale Veröffentlichungsnummer: WO 2004/015046

(56) Entgegenhaltungen:
- WO-A-00/09163
- DE-A- 10 143 534
- US-A1- 2001 025 113
- US-B1- 6 410 761
- US-B1- 6 414 171

## Beschreibung

### Gebiet der Erfindung

Die Erfindung befindet sich auf dem Gebiet der Fettsäuren und betrifft ein neues Verfahren zur Herstellung von konjugierter Linolsäure durch Verseifung ihrer Ester und Neutralisation mit Phosphorsäure.

### Stand der Technik

Mehrfach ungesättigte Linolsäuren mit konjugierten Doppelbindungen, die unter der Bezeichnung "CLA" (conjugated linoleic acid) im Handel sind, gehören zu den essentiellen Fettsäuren für Mensch und Tier und werden daher als Lebensmittelzusatzstoffe eingesetzt. Üblicherweise geht man zur Herstellung von konjugierter Linolsäure von Triglyceriden aus, die über einen hohen Anteil an - üblicherweise nicht-konjugierter - Linolsäure verfügen, wie beispielsweise Distel- oder Sonnenblumenöl. Die Triglyceride werden in Gegenwart von basischen Katalysatoren isomerisiert und gleichzeitig verseift. Von Nachteil dabei ist, dass die Verseifung zum einen eine Menge unerwünschter Abfallstoffe liefert und zudem hohe Mengen an Alkalien erforderlich sind, was rasch zu Korrosion in den Reaktoren führen kann. Um dies zu vermeiden, geht man in neuerer Zeit vorzugsweise von den Linolsäurealkylestern aus, die zunächst zu den CLA-Estern isomerisiert und dann verseift werden. Bei diesem Verfahren muß man jedoch häufig in Kauf nehmen, dass die Kesselauslastung sehr gering ist. Durch hohe Wassermengen, geringe Ausbeuten, sowie unerwünschte Nebenprodukte wird die Rentabilität dieses Verfahrens erheblich einschränkt.

Das Dokument US 2001/0025113 beschreibt die Herstellung von CLA.

Die Aufgabe der vorliegenden Erfindung hat folglich darin bestanden, ein Verfahren zur Herstellung von konjugierter Linolsäure zur Verfügung zu stellen, das sich durch eine hohe Rentabilität auszeichnet und zu einem Endprodukt in hoher Ausbeute und guter Reinheit führt.

### Beschreibung der Erfindung

Gegenstand der Erfindung ist ein Verfahren zur Herstellung von konjugierter Linolsäure, bei dem man
(a) Linolsäureniedrigalkylester in Gegenwart von Alkalialkoholaten isomerisiert
(b) die dann konjugierten Linolsäureniedrigalkylester in Gegenwart von Alkalilauge mit Wasser verseift und
(c) das Verseifungsprodukt mit Phosphorsäure neutralisiert

Überraschenderweise wurde gefunden, dass eine Neutralisation eines Verseifungsproduktes konjugierter Linolsäureniedrigalkylester mit Phosphorsäure zu einer sehr guten Kesselauslastung bei der Herstellung konjugierter Fettsäuren führt. Eine Rückveresterung nach der Verseifung wird minimiert, so dass während der Herstellung wenig unerwünschte Nebenprodukte entstehen. Man erhält nach der Neutralisation mit Phosphorsäure und anschließender Phasentrennung ein Endprodukt in hoher Ausbeute und hoher Reinheit auf Grund des geringen Ester-gehaltes.

### Linolsäureniedrigalkylester

Als Ausgangsstoffe für das erfindungsgemäße Verfahren dienen Linolsäureniedrigalkylester, die vorzugsweise der Formel (I) folgen,

**R¹CO-OR²** **(I)**

in der R¹CO für den Acylrest einer Linolsäure und R² für einen linearen oder verzweigten Alkylrest mit 1 bis 5 Kohlenstoffatomen steht. Insbesondere werden konjugierte Linolsäuremethyl- und/oder -ethylester eingesetzt.

### Isomerisierung

Die Isomerisierung der Linolsäureniedrigalkylester wird mit Alkalialkoholaten unter Begasung mit Inertgas bei Temperaturen im Bereich von 90 bis 150°C, vorzugsweise 100 bis 130 °C und besonders bevorzugt 105 bis 125 °C durchgeführt.

In einer bevorzugten Ausführungsform werden Alkalialkoholate mit 1 bis 10 C-Atomen als Basen während der Isomerisierung verwendet, besonders bevorzugt werden Kaliummethanolat, Kaliumethanolat oder Kalium-t-butylat eingesetzt.

### Verseifung

Die Verseifung der isomerisierten Linolsäureniedrigalkylester mit wässrigen Alkalilaugen erfolgt bei Temperaturen im Bereich von 40 bis 90 °C, vorzugsweise 60 bis 80 °C und besonders bevorzugt 65 bis 75 °C. Sie wird bis zu einem Spaltgrad von 80 bis 100 Gew. %, vorzugsweise größer 98% durchgeführt.

### Neutralisation

Der für die ökonomische Durchführung (hohe Kesselauslastung) wesentlichste Schritt im Verfahren ist die Neutralisation mit Phosphorsäure und die Aufarbeitung durch Phasentrennung, wobei die entstandenen Salze in der wässrigen Phase gelöst bleiben. Die Neutralisation mit Phosphorsäure wird vorzugsweise in einer Konzentration von 75- 85 Gew.% eingesetzt. Dabei wird auch hier bei Temperaturen im Bereich von 40 bis 90 °C, vorzugsweise 60 bis 80 °C und besonders bevorzugt 65 bis 75 °C gearbeitet. Vor der Neutralisation kann der Ansatz durch Zufügen von Wasser auf die gewünschte Viskosität eingestellt werden.

### Aufarbeitung

Im Anschluss an die Neutralisation wird bei 50 bis 100°C, vorzugsweise 70 bis 90 °C eine Phasentrennung durchgeführt. Die Phasentrennung wird optimiert durch erhöhte Temperaturen. Danach wird eine Trocknung im Vakuum bei über 100°C, vorzugsweise über 110 °C angeschlossen.

### Beispiel

### Herstellung von konjugierter Linolsäure aus Linolsäureethylester

In einen beheizbaren Kolben wurden 1190 g Linolsäureethylester vorgelegt und unter Rühren, Stickstoffbegasung und kontinuierlichem Abdestillieren von Ethanol, wurden bei einer Temperatur von 110°C 60 g Kaliumethanolat (32 Gew.%) zugefügt. Nach Zugabe von 190 g Wasser wurden bei einer Temperatur von 70°C 1070 g einer 25 Gew.%ige Kaliumhydroxid-Lösung zur Verseifung in den Kolben gepumpt. Unter Rühren wurden danach wiederum 770 g Wasser zugefügt und bei einer Temperatur von 70 °C 510 g Phosphorsäure (85 Gew.%) zur Neutralisation zugefügt. Danach erfolgte der Austrag von Waschwasser und die nachfolgende Phasentrennung bei einer Temperatur von 70 bis 90 °C .

Die auf diese Weise gewonnene konjugierte Linolsäure wies die folgenden Kenndaten auf:

| | |
|---|---|
| Säurezahl: | 199 |
| Verseifungszahl: | 200 |
| OH-Zahl: | 4,9 |
| Jodzahl: | 162 |
| Unverseifbare Anteile: | 0,1 % |

## Patentansprüche

1. Verfahren zur Herstellung von konjugierter Linolsäure, bei dem man
(a) Linolsäureniedrigalkylester in Gegenwart von Alkalialkoholaten isomerisiert
(b) die dann konjugierten Linolsäureniedrigalkylester in Gegenwart von Alkalilauge mit Wasser verseift und
(c) das Verseifungsprodukt mit Phosphorsäure neutralisiert.

2. Verfahren nach Anspruch 1, **dadurch gekennzeichnet, dass** man Linolsäureniedrigalkylester der Formel **(I)** einsetzt,
**R¹CO-OR²** **(I)**
in der R¹CO für den Acylrest einer Linolsäure und R² für einen linearen oder verzweigten Alkylrest mit 1 bis 5 Kohlenstoffatomen steht.

3. Verfahren nach den Ansprüchen 1 und/oder 2, **dadurch gekennzeichnet, dass** man die Isomerisierung bei Temperaturen im Bereich von 90 bis 150 °C durchführt.

4. Verfahren nach mindestens einem der Ansprüche 1 bis 3, **dadurch gekennzeichnet, dass** man die Verseifung bei Temperaturen im Bereich von 40 bis 90 °C durchführt....

5. Verfahren nach mindestens einem der Ansprüche 1 bis 4, **dadurch gekennzeichnet, dass** man die Verseifung bis zu einem Spaltgrad von 80 bis 100 Gew.-% durchführt.

6. Verfahren nach mindestens einem der Ansprüche 1 bis 5, **dadurch gekennzeichnet, dass** man die Neutralisation mit Phosphorsäure bei Temperaturen im Bereich von 50 bis 90°C durchführt.

7. Verfahren nach mindestens einem der Ansprüche 1 bis 6, **dadurch gekennzeichnet, dass** man die der Neutralisation folgende Phasentrennung bei Temperaturen im Bereich von 50 bis 100°C durchführt.

## Claims

1. A process for the production of conjugated linoleic acid, in which
(a) linoleic acid lower alkyl esters are isomerized in the presence of alkali metal alcoholates,
(b) the now conjugated linoleic acid lower alkyl esters are saponified with water in the presence of lye and
(c) the saponification product is neutralized with phosphoric acid.

2. A process as claimed in claim 1, **characterized in that** linoleic acid lower alkyl esters corresponding to formula **(I)**:
**R¹CO-OR²** **(I)**
where R¹CO is the acyl group of a linoleic acid and R² is a linear or branched alkyl group containing 1 to 5 carbon atoms,
are used.

3. A process as claimed in claims 1 and/or 2, **characterized in that** the isomerization step is carried out at temperatures of 90 to 150°C.

4. A process as claimed in at least one of claims 1 to 3, **characterized in that** the saponification step is carried out at temperatures of 40 to 90°C.

5. A process as claimed in at least one of claims 1 to 4, **characterized in that** the saponification step is continued to a cleavage level of 80 to 100% by weight.

6. A process as claimed in at least one of claims 1 to 5, **characterized in that** the neutralization step is carried out with phosphoric acid at temperatures of 50 to 90°C.

7. A process as claimed in at least one of claims 1 to 6, **characterized in that** the phase separation following the neutralization step is carried out at temperatures of 50 to 100°C.

## Revendications

1. Procédé de préparation d'acide linoléique conjugué, selon lequel
(a) on isomérise des esters alkyliques à faible poids moléculaire d'acide linoléique en présence d'alcoolates alcalins
(b) on saponifie les esters alkyliques à faible poids moléculaire d'acide linoléique alors conjugués en présence de lessive alcaline avec de l'eau et
(c) on neutralise le produit de la saponification avec de l'acide phosphorique.

2. Procédé selon la revendication 1,
**caractérisé en ce qu'**
on utilise des esters alkyliques à faible poids moléculaire d'acide linoléique de formule (I)
R¹CO-OR² (I)
dans laquelle R¹CO représente le radical acyle d'un acide linoléique et R² un radical alkyle linéaire ou ramifié comportant 1 à 5 atomes de carbone.

3. Procédé selon les revendications 1 et/ou 2,
**caractérisé en ce qu'**
on réalise l'isomérisation à des températures de l'ordre de 90 à 150 °C.

4. Procédé selon au moins l'une des revendications 1 à 3,
**caractérisé en ce qu'**
on réalise la saponification à des températures de l'ordre de 40 à 90 °C.

5. Procédé selon au moins l'une des revendications 1 à 4,
**caractérisé en ce qu'**
on réalise la saponification jusqu'à un degré de clivage de 80 à 100 % en poids.

6. Procédé selon au moins l'une des revendications 1 à 5,
**caractérisé en ce qu'**
on réalise la neutralisation avec de l'acide phosphorique à des températures de l'ordre de 50 à 90 °C.

7. Procédé selon au moins l'une des revendications 1 à 6,
**caractérisé en ce qu'**
on réalise la séparation de phases suivant la neutralisation à des températures de l'ordre de 50 à 100 °C.
